# EUROPÄISCHE PATENTANMELDUNG

(11) **EP 2 439 263 A1**
(43) Veröffentlichungstag der Anmeldung: **11.04.2012**
(21) Anmeldenummer: 11183912.2
(22) Anmeldetag: 05.10.2011
(51) Int. Cl.: C12N 5/00, A61L 27/38

(54) **Flexibles Zellkulturträgermaterial mit einer porösen dreidimensionalen Struktur**

(30) Priorität: 05.10.2010 DE 102010041985
(71) Anmelder: KET Kunststoff- und Elasttechnik GmbH Liegau-Augustusbad, 01454 Radeberg (DE)
(72) Erfinder: Wenske, Günther, 01454 Radeberg (DE); Böttcher, Gunter, 01561 Würschnitz (DE); Winkelmann, Claudia, 08412 Werdau OT Steinpleis (DE)
(74) Vertreter: Uhlemann, Henry

(57) **Zusammenfassung**

Die Erfindung betrifft ein Verfahren zur Herstellung eines flexiblen Zellkulturträgermaterials mit einer porösen dreidimensionalen Struktur, ein flexibles Zellkulturträgermaterial, Verfahren zur Kultivierung von Zellen auf einem flexiblen Zellkulturträgermaterial und die Verwendung eines flexiblen Zellkulturträgermaterials zur *in vitro* Kultivierung von Zellen.

Das erfindungsgemäße Verfahren umfasst die Schritte:
a) Bereitstellung eines porösen dreidimensionalen Formkörpers mittels Rapid Prototyping. Dabei ist der Formkörper offenporig und weist ein interkonnektierendes Porensystem auf. Der Formkörper enthält mindestens einen elastischen, inerten Kunststoff mit einer hydrophoben Oberfläche. Der dreidimensionale Formkörper ist ein dreidimensionales Gitter.
b) Behandlung der Oberfläche des porösen dreidimensionalen Formkörpers, so dass die hydrophobe Oberfläche des im Formkörper enthaltenen elastischen, inerten Kunststoffs in eine hydrophile Oberfläche umgewandelt wird.
c) Aufbringen einer bioaktiven Schicht auf die hydrophile Oberfläche des elastischen, inerten Kunststoffs des Formkörpers durch Kontaktieren des Formkörpers mit einer Flüssigkeit, die mindestens ein bioaktives Protein enthält.

## Beschreibung

Die Erfindung betrifft ein Verfahren zur Herstellung eines flexiblen Zellkulturträgermaterials mit einer porösen dreidimensionalen Struktur, ein flexibles Zellkulturträgermaterial, Verfahren zur Kultivierung von Zellen auf einem flexiblen Zellkulturträgermaterial und die Verwendung eines flexiblen Zellkulturträgermaterials zur *in vitro* Kultivierung von Zellen. Die Erfindung findet Anwendung in den Bereichen der Medizintechnik und der Biotechnologie.

Bei der *in vitro* Kultivierung von Zellen werden nicht-adhärente Zellen als Suspensionskultur gehalten während adhärente Zellen einen Träger benötigen, an den sich die Zellen anheften können. Im einfachsten Fall wird ein Zellkulturgefäß eingesetzt, an dessen Oberfläche sich die adhärenten Zellen anheften (adhärieren). Da die Anheftung der Zellen durch bestimmte Oberflächenproteine begünstigt wird, werden die Zellkulturgefäße meist mit Proteinen der extrazellulären Matrix oder anderen strukturverwandten Proteinen, wie Poly-L-Lysin, beschichtet, um das Adhärieren der Zellen zu erleichtern. Die Adhäsion von Zellen auf einer Oberfläche wird hierin auch als "Besiedlung der Oberfläche" bezeichnet.

Adhärente Zellen entstammen ursprünglich einem bestimmten Gewebe, beispielsweise Leber, Niere, Muskeln, Nerven, Endothel oder Epithel. Werden diese Zellen in Langzeit- oder Dauerkultur gehalten, können die Zellen bei entsprechenden Voraussetzungen dreidimensionale Strukturen ausbilden, die dem *in vivo* vorliegenden Gewebe ähneln und können so beispielsweise als Gewebemodelle dienen. Eine entscheidende Voraussetzung, damit die Zellen gewebeähnliche Strukturen ausbilden, ist es, ein dreidimensionales Wachstum der Zellen zu generieren. Durch die oben genannte Kultivierung auf der Oberfläche von Zellkulturgefäßen ist jedoch lediglich ein zweidimensionales Wachstum möglich, da die Zellen ausschließlich auf der Oberfläche des Zellkulturgefäßes haften können.

Um ein dreidimensionales Wachstum der Zellen zu ermöglichen, ist eine dreidimensionale Trägerstruktur erforderlich. Um dreidimensionale Trägerstrukturen zu erzeugen, besteht die Möglichkeit, ebene Oberflächen zu strukturieren. Dies kann beispielsweise durch Laserabtragung, über Ätztechniken, durch werkzeuggebundene Oberflächentexturierung, die eine strukturierte ebene Oberfläche erzeugen oder durch Rapid Prototyping geschehen.

Alternativ dazu können direkt dreidimensionale Trägerstrukturen hergestellt werden. Die Trägerstruktur muss porös sein, so dass die Zellen in die Trägerstruktur einwachsen können und sie muss eine Oberfläche aufweisen, an der die Zellen adhärieren können. Zur Herstellung solcher dreidimensionalen porösen Trägerstrukturen sind verschiedene Verfahren bekannt. Beispiele sind textiltechnische Verfahren, wie Stricken, Sticken, Weben, Wirken, Flocktechnologien oder die Verwendung von porösen Vliesstoffen. Weitere bekannte Verfahren zur Herstellung dreidimensionaler Strukturen sind die Verfahren der Gefriertrocknung, das Vergießen und Verfestigen von Polymerlösungen, Sintern, Schäumen oder die Technologie des Rapid Prototyping.

Zur Herstellung von dreidimensionalen Trägerstrukturen werden bisher sowohl resorbierbare als auch nicht-resorbierbare Materialien eingesetzt. Resorbierbare Materialien werden während der Besiedelung durch Zellen verstoffwechselt und werden so mit der Zeit abgebaut. Nicht-resorbierbare Materialien werden nicht verstoffwechselt und bleiben während der Kultivierung dauerhaft erhalten. Derzeit in dreidimensionalen Trägerstrukturen eingesetzte resorbierbare Materialien sind biologische Polymere, wie beispielsweise Alginate, Agar-Agar, Gelatine oder Kollagene, oder synthetische Materialien, wie Poly-L-Lactid, Polyglycolid oder Keramiken aus Calciumphosphaten. Entsprechende Trägerstrukturen aus Hydrogelen werden beispielsweise durch Rapid Prototyping hergestellt.

Diese Trägerstrukturen sind hochgradig bioaktiv, d.h. dass sie durch die Zellen gut und effektiv besiedelbar sind. Durch den Abbau der Trägerstrukturen durch Resorption ist jedoch keine dauerhafte Gerüststruktur für die Zellen gegeben, die bei einer dauerhaften Kultivierung erforderlich ist. Weiterhin sind die eingesetzten biologischen Polymere in den meisten Fällen nach der Benetzung mit Zellkulturmedium nicht formstabil. Kritisch ist die Sterilisierung dieser Materialien, da insbesondere die biologischen Materialien unter Sterilisationsbedingungen denaturieren. Ein Einsatz im unsterilen Zustand birgt jedoch die Gefahr der Kontamination und ist somit für eine dauerhafte Kultivierung nur bedingt geeignet.

Zellkulturträgerstrukturen aus nicht-resorbierbaren Materialien enthalten beispielsweise synthetische Polymere, wie Polyurethan, Polyethylen, Polystyrol oder Silikone. Zur erfolgreichen Besiedlung der Zellen ist dabei entscheidend, dass die Porosität für den jeweiligen Zelltyp angemessen ist, so dass die Poren nicht zu klein sind, und so ein Einwachsen der Zellen verhindern, aber andererseits auch nicht zu groß sind, so dass ein ausreichender Kontakt zwischen den Zellen sichergestellt ist. Um eine effektive Kultivierung der adhärierenden Zellen zu ermöglichen, ist eine Behandlung der Oberfläche erforderlich.

Der Einsatz von Zellkulturträgermaterialien aus Silikonen beschränkt sich bisher auf zweidimensionale Oberflächen oder als dreidimensionale Oberfläche in der Form strukturierter ebener Oberflächen. Dafür wird die hydrophobe Oberfläche des Silikons durch Plasmabehandlung oder Beflammung in eine hydrophile Schicht umgewandelt. Diese Verfahren sind typische "line of sight" Verfahren, bei denen eine homogene Behandlung komplexer dreidimensionaler Strukturen nicht immer möglich ist, da Hinterschneidungen des Materials nicht behandelt werden können.

Silikon ist ein Material, welches hervorragend für den Aufbau von dreidimensionalen Strukturen durch Rapid Prototyping geeignet ist (Landers R, Dissertation, Albert-Ludwigs-Universität Freiburg, 2004). Durch seine hydrophobe Oberfläche wird es jedoch bisher nur in den Formen als strukturierte plane Oberfläche als Zellkulturträgermaterial eingesetzt. Der Einsatz komplexer dreidimensionaler Strukturen aus Silikon als Zellkulturträgermaterial ist bisher nicht möglich.

Aufgabe der Erfindung ist es, ein dreidimensionales Zellkulturträgermaterial bereitzustellen, das porös und elastisch ist und ein Anwachsen der Zellen in der dreidimensionalen Struktur erlaubt. Das Zellkulturträgermaterial, insbesondere dessen Porosität, soll je nach Anwendungszweck ausgestaltbar sein.

Die Aufgabe wird erfindungsgemäß gelöst durch ein Verfahren zur Herstellung eines elastischen Zellkulturträgermaterials mit einer porösen dreidimensionalen Struktur, welches zur *in vitro* Kultivierung von Zellen geeignet ist und ein dreidimensionales Wachstum von Zellen ermöglicht. Das erfindungsgemäße Verfahren ist durch die folgenden Schritte gekennzeichnet:
a) Bereitstellung eines porösen dreidimensionalen Formkörpers mittels Rapid Prototyping. Der dreidimensionale Formkörper ist ein dreidimensionales Gitter, das vorzugsweise aus mindestens zwei übereinanderliegenden parallelen Lagen aus jeweils mindestens zwei länglichen Körpern besteht, wobei die Lagen in einem Winkel von 1 bis 90° zueinander versetzt angeordnet sind, so dass sich die länglichen Körper der unterschiedlichen Lagen kreuzen. Der dreidimensionale Formkörper ist offenporig und weist ein interkonnektierendes Porensystem auf. Der Formkörper enthält mindestens einen elastischen, inerten Kunststoff mit einer hydrophoben Oberfläche.
b) Behandlung der Oberfläche des porösen dreidimensionalen Formkörpers, so dass die hydrophobe Oberfläche des im Formkörper enthaltenen elastischen, inerten Kunststoffs in eine hydrophile Oberfläche umgewandelt wird.
c) Aufbringen einer bioaktiven Schicht auf die hydrophile Oberfläche des elastischen, inerten Kunststoffs des Formkörpers durch Kontaktieren des Formkörpers mit einer Flüssigkeit, die mindestens ein bioaktives Protein enthält.

Basis des Zellkulturträgermaterials ist der poröse dreidimensionale Formkörper, der so ausgestaltet ist, dass durch die Dimensionierung des Porensystems optimale räumliche Wachstumsbedingungen für die zu kultivierenden Zellen herrschen. Durch die Behandlung der Oberfläche des dreidimensionalen Formkörpers wird eine bioaktive Oberfläche auf dem Formkörper aufgebracht, welche mindestens ein bioaktives Protein enthält und durch die ein Adhärieren der zu kultivierenden Zellen ermöglicht wird. Unter einem erfindungsgemäßen Zellkulturträgermaterial wird daher der bioaktiv beschichtete poröse dreidimensionale Formkörper verstanden. Folglich besitzt auch das erfindungsgemäße Zellkulturträgermaterial eine poröse dreidimensionale Struktur.

Zellkulturträgermaterialien, die mit einem erfindungsgemäßen Verfahren hergestellt werden, sind elastisch. Um die Elastizität des gesamten Zellkulturträgermaterials zu gewährleisten, enthält der dreidimensionale Formkörper ausschließlich elastische Materialien (Feststoffe). Dabei enthält der Formkörper mindestens einen elastischen, inerten Kunststoff. Unter inert ist in diesem Sinne zu verstehen, dass der Kunststoff nicht oder nur in verschwindend geringem Maße mit dem Zellkulturmedium reagiert und keine Auswirkung auf das Wachstum der zu kultivierenden Zellen, insbesondere keine toxische Wirkung, hat. Es handelt sich somit bei dem inerten Kunststoff um einen nicht-resorbierbaren Kunststoff. Dadurch wird gewährleistet, dass das Zellkulturträgermaterial einerseits flexibel ist, was eine einfache Handhabung und ein einfaches Entfernen der Zellen nach der Kultivierung ermöglicht. Gleichzeitig wird der dreidimensionale Formkörper nicht von den Zellen verstoffwechselt und nicht vom Zellkulturmedium angegriffen und ist somit wiederverwendbar.

Der poröse dreidimensionale Formkörper, der in erfindungsgemäßen Verfahren eingesetzt wird, wird durch Verfahren des Rapid Prototyping hergestellt. Dadurch wird es erreicht, ein definiertes Porensystem herzustellen, dessen Porengröße und Porenverteilung ein optimales Wachstum der zu kultivierenden Zellen ermöglicht.

Die Form und Dimension des dreidimensionalen Formkörpers wird dabei so ausgewählt, dass ein dreidimensionales Wachstum gefördert wird, insbesondere wird dabei die Porengröße des Formkörpers so eingestellt, dass die Porengröße die durchschnittliche Größe (vorzugsweise Durchmesser) der zu kultivierenden Zellen um nicht mehr als das Dreifache, vorzugsweise nicht mehr als das Doppelte überschreitet. Unter Porengröße wird dabei im Sinne der Erfindung bei runden Poren der Durchmesser der Poren verstanden. Bei ungleichmäßig gebildeten Poren wird dabei als Porendurchmesser im Sinne der Erfindung der Mittelwert zwischen dem kleinsten und größten Durchmesser einer Pore verstanden.

Dadurch wird vermieden, dass der dreidimensionale Formkörper zu kleine Poren enthält, in welche die Zellen bei der Kultivierung nicht einwachsen können. Weiterhin wird gewährleistet, dass keine zu großen Poren enthalten sind, die dazu führen, dass die Zellen bei der Besiedlung durch die Poren fallen können und sich vermehrt am Boden des Kulturgefäßes ansammeln würden und das Zellkulturträgermaterial ineffizient besiedelt würde. Durch einen dreidimensionalen Formkörper, der durch Rapid Prototyping hergestellt wird, und dessen Porengröße daher gezielt so ausgewählt werden kann, dass sie optimale Wachstumsbedingungen für die jeweiligen Zellen bietet, wird somit ein besonders effektives dreidimensionales Wachstum der Zellen ermöglicht.

Verfahren des Rapid Prototyping sind aus dem Stand der Technik bekannt. Bei diesen Verfahren werden auf dem Computer vorhandene Daten direkt zur Herstellung eines Formkörpers umgesetzt. Die dreidimensionalen Formkörper, die in einem erfindungsgemäßen Verfahren zum Einsatz kommen, werden bevorzugt durch Verfahren des 3D-Printing hergestellt, bei dem eine schnell aushärtende viskose Masse Schicht für Schicht durch Spritzen in Form gebracht wird. Es ist mittels Rapid Prototyping auch möglich, dreidimensionale Formkörper aus unterschiedlichen Materialien herzustellen. So enthält der dreidimensionale Formkörper mindestens einen elastischen, inerten Kunststoff und ggf. auch weitere Materialien. Der elastische, inerte Kunststoff ist vorzugsweise zu mindestens 50 Volumen-%, weiter bevorzugt zu mindestens 75 Volumen-% in der dreidimensionalen Struktur enthalten. Bevorzugt ist der elastische Kunststoff ausgewählt aus Silikon, Polystytol, Polypropylen und Polyurethan, besonders bevorzugt Silikon. Silikone, die zur Herstellung der Formkörper für erfindungsgemäße Zellkulturträgermaterialien eingesetzt werden sind bevorzugt elastische Silikonkautschuke, die Poly(organo)siloxane enthalten.

Der dreidimensionale Formkörper enthält ggf. weitere Materialien, wobei die Materialien so ausgewählt sind, dass sie sich für eine Anwendung in Verfahren des Rapid Prototyping eignen. Vorzugsweise sind die weiteren Materialien schnell aushärtende, elastische Kunststoffe, die nicht identisch mit dem elastischen, inerten Kunststoff ist, der mindestens enthalten sein muss.

Für die Herstellung erfindungsgemäßer Zellkulturträgermaterialien werden solche Formkörper verwendet, die offenporig sind und ein interkonnektierendes Porensystem aufweisen. Durch die Offenporigkeit, also die Öffnung des Porensystems nach außen, wird das Eindringen und Einwachsen der Zellen in das Innere des Formkörpers ermöglicht. Das interkonnektierende Porensystem ist notwendig, damit alle Poren vom Zellkulturmedium durchströmt und von den Zellen besiedelt werden können. Die Größe der Poren des dreidimensionalen Formkörpers beträgt dabei vorzugsweise zwischen 0,4 mm und 1 mm.

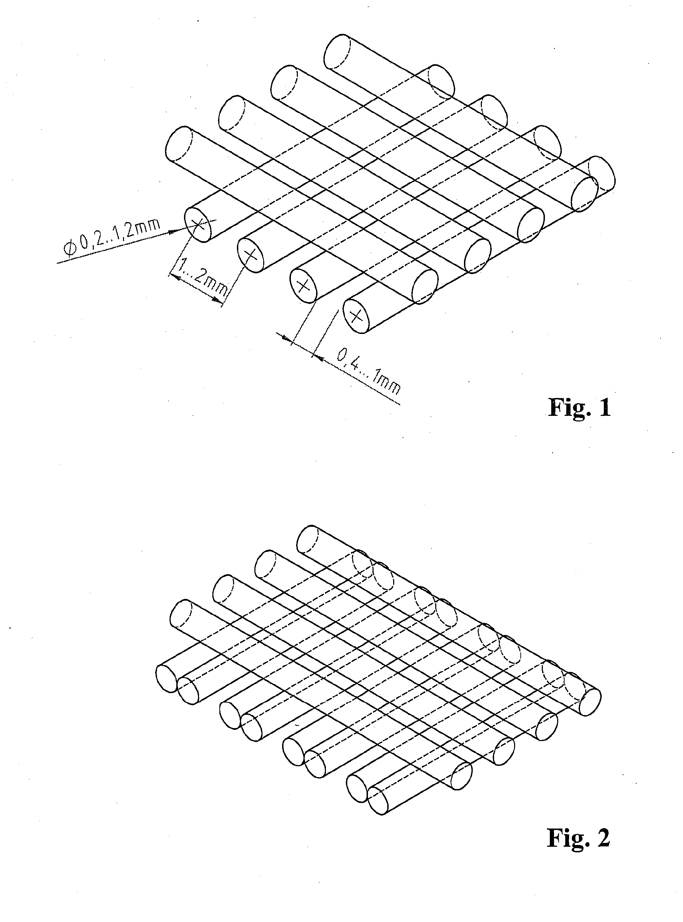

Der poröse dreidimensionale Formkörper ist als dreidimensionales Gitter ausgebildet. Das Gitter umfasst vorzugsweise mindestens zwei übereinanderliegende parallele Lagen aus länglichen Körpern, insbesondere aus länglichen zylinderförmigen Körpern, wobei die Lagen in einem Winkel von 1 bis 90° zueinander versetzt angeordnet sind, so dass sich die länglichen Körper der unterschiedlichen Lagen kreuzen. Die Breite der länglichen Körper bzw. bei länglichen Körpern mit einer runden Querschnittsfläche deren Durchmesser, beträgt dabei vorzugsweise zwischen 0,2 mm und 1,2 mm. Die Abstände der länglichen Körper betragen vorzugsweise 1 mm bis 2 mm, gemessen von der Mitte der länglichen Körper. Die Porengröße von einer solchen dreidimensionalen Gitterstruktur entspricht im Sinne der Erfindung der Abstand der länglichen Körper einer Lage (gemessen von der Außenseite eines länglichen Körpers, siehe dazu Fig. 1) und beträgt vorzugsweise 0,4 mm bis 1 mm.

Die Herstellung dreidimensionaler Gitter durch Rapid Prototyping ermöglicht es, auf besonders einfachem Weg dreidimensionale Gitter, die bevorzugt mehrere Materialien enthalten, herzustellen. Dabei ist mindestens eine weitere Lage aus länglichen Körpern im Gitter enthalten ist, die aus einem weiteren elastischen Material besteht oder es ist mindestens eine Lage enthalten, die sowohl längliche Körper aus einem elastischen, inerten Kunststoff als auch längliche Körper aus weiteren elastischen Material enthält.

In einer Ausführungsform sind daher dreidimensionale Gitter aus Silikon bevorzugt. In einer anderen Ausführungsform sind dreidimensionale Gitter bevorzugt, die Silikon und mindestens ein Protein der extrazellulären Matrix im Gitter enthalten, davon besonders bevorzugt Kollagen. In einer weiteren Ausführungsform sind dreidimensionale Gitte bevorzugt, die Silikon enthalten und mindestens einen weiteren elastischen, inerten Kunststoff, der nicht Silikon ist.

Damit es ermöglicht wird, die hydrophobe Oberfläche des dreidimensionalen Formkörpers mit Zellen zu besiedeln, wird diese zunächst durch einen Verfahrensschritt in eine hydrophile Oberfläche umgewandelt. Anschließend wird darauf eine bioaktive Schicht aufgebracht, die mindestens ein bioaktives Protein enthält. An diese bioaktive Schicht adhärieren die Zellen nach der Besiedlung des Zellkulturträgermaterials.

Zur Umwandlung der hydrophoben Oberfläche in eine hydrophile Oberfläche wird entweder eine Plasmabehandlung, eine Beflammung oder eine nasschemische Behandlung in einer Lösung mit MSA-Copolymeren durchgeführt.

Plasmabehandlungsmethoden, wie sie aus dem Stand der Technik bekannt sind, sind für die Anwendung in einem erfindungsgemäßen Verfahren geeignet. Der dreidimensionale Formkörper wird vor der Plasmabehandlung vorzugsweise gereinigt, insbesondere durch eine Spülung in einem Lösungsmittel, wie beispielsweise Ethanol. Die Plasmabehandlung wird vorzugsweise unter Schutzgasatmosphäre durchgeführt. Bevorzugt wird Argon als Schutzgas eingesetzt. Die Dauer der Behandlung beträgt vorzugsweise zwischen 30 Sekunden und 10 Minuten, besonders bevorzugt zwischen 200 und 400 Sekunden.

Alternativ zu einer Plasmabehandlung erfolgt bevorzugt eine Beflammung des dreidimensionalen Formkörpers. Dafür wird der Formkörper mit einem Flammstrahl aus einem entzündlichen Gas kontaktiert. Dafür geeignete Gasgemische sind insbesondere Propan-Butan-Gase. Zur Beflammung der dreidimensionalen Formkörper wird dabei zunächst die Flamme entzündet und der Formkörper kurz mit der Flamme kontaktiert. Die Kontaktzeit beträgt vorzugsweise weniger als eine Sekunde. Der dreidimensionale Formkörper darf nicht so lang beflammt werden, dass ein Schmelzen des Formkörpers eintritt. Auch vor der Beflammung erfolgt vorzugsweise ein separater Reinigungsschritt, der analog zu der Reinigung vor der Plasmabehandlung durchgeführt wird. Besonders bevorzugt werden dreidimensionalen Formkörper in einem erfindungsgemäßen Verfahren beflammt, um die hydrophobe Oberfläche zu hydrophilieren.

Als Alternative zu Plasmabehandlung und Beflammung wird vorzugsweise eine nasschemische Behandlung des dreidimensionalen Formkörpers vorgenommen, durch die dessen hydrophobe Oberfläche in eine hydrophile Oberfläche umgewandelt wird. Vorteil einer nasschemischen Behandlung ist, dass auch komplexe dreidimensionale Formkörper oberflächenmodifiziert werden können. Grundsätzlich sind dafür aus dem Stand der Technik bekannte nasschemische Behandlungsverfahren geeignet, mit denen eine hydrophobe Oberfläche hydrophiliert werden kann. Vor der nasschemischen Behandlung in einem erfindungsgemäßen Verfahren wird keine Plasmabehandlung oder Beflammung durchgeführt. Die nasschemische Behandlung wird direkt auf der hydrophoben Oberfläche des dreidimensionalen Formkörpers vorgenommen.

Bevorzugt werden zur nasschemischen Behandlung von dreidimensionalen Formkörpern aus Silikon Copolymere und/oder Terpolymere aus Maleinsäureanhydrid und mindestens einem Vinylmonomer, insbesondere Allyltrimethylsilan (hierin vereinfacht als "MSA-Copolymere" bezeichnet, wobei auch die Terpolymere von diesem Begriff umfasst sind), sowie niedermolekulare Polysiloxane mit Aminogruppen (also aminogruppenhaltige Silikonflüssigkeiten) eingesetzt.

Die Polysiloxane enthalten vorzugsweise 2-10, insbesondere 2-6 Aminogruppen je Molekül. Die Polysiloxane weisen bevorzugt ein Molekulargewicht von 3000 - 10000 g/mol, insbesondere von 3000 - 5000 g/mol auf. Die Viskosität der Polysiloxane ist niedrig und beträgt vorzugsweise maximal 200 centistoke, bevorzugt maximal 100 centistoke, insbesondere zwischen 30 und 100 centistoke (kinematische Viskosität im Kapillarviskosimeter). Die Polysiloxane liegen in konzentrierter Form oder in einem Lösungsmittel vor, insbesondere in Aceton. Bevorzugte Polysiloxane sind Silikonöle, wie Epoxy-Silikonöle.

Die MSA-Copolymere weisen bevorzugt ein Molekulargewicht von 1000 - 20000 g/mol auf, bevorzugt von 4000 - 10000 g/mol. Die MSA-Copolymere liegen bevorzugt in einem organischen Lösungsmittel, vorzugsweise Aceton, Tetrahydrofuran, Methylethylketon oder deren Mischungen, insbesondere in Aceton vor. Die Konzentration der MSA-Copolymere in dem Lösungsmittel beträgt vorzugsweise 5-20 Gewichts-%, insbesondere 8-15 Gewichts-%.

Zur Anbindung der MSA-Copolymere an die Silikonoberfläche wird der dreidimensionale Formkörper zunächst in einem ersten Tauchvorgang, bei dem der Formkörper in die konzentrierten Polysiloxane oder die Lösung mit Polysiloxanen getaucht wird, behandelt. Der Tauchvorgang wird vorzugsweise für mehrere Minuten, insbesondere für 1-30 Minuten durchgeführt. Anschließend wird der Formkörper in einem zweiten Tauchvorgang in die Lösung mit MSA-Copolymeren eingetaucht, wodurch die MSA-Copolymere eine Bindung mit den Aminogruppen der Polysiloxane eingehen. Vor der Inkubation des Formkörpers in der Lösung mit MSA-Copolymeren werden diese nach der Inkubation in der Polysiloxanlösung vorzugsweise gespült, um die überschüssigen Polysiloxane zu entfernen und anschließend getrocknet. Die Inkubation des Formkörpers mit den MSA-Copolymeren wird vorzugsweise zunächst unter Schütteln für mehrere Minuten, insbesondere mindestens 2 Minuten, besonders bevorzugt mindestens 5 Minuten durchgeführt. Unabhängig davon, ob zunächst eine kurze Inkubation unter Schütteln erfolgte, wird der Formkörper zur Inkubation mit den MSA-Copolymeren für mehrere Minuten, insbesondere mindestens 30 min, in die Lösung mit den MSA-Copolymeren getaucht. Die Inkubation kann vorzugsweise bei erhöhter Temperatur vorgenommen werden, insbesondere bei mindestens 100°C. Im Anschluss an die Inkubation wird der dreidimensionale Formkörper getrocknet. Dies erfolgt vorzugsweise bei erhöhter Temperatur, insbesondere mindestens 30°C, besonders bevorzugt bei 50 °C und mehr, ganz besonders bevorzugt von 50°C bis 100°C.

An der Bindung der MSA-Copolymere an die Aminogruppen der Polysiloxane nimmt nur ein geringer Anteil der Säureanhydridgruppen teil. Die übrigen Säureanhydridgruppen der MSA-Copolymere stehen für die Anbindung weiterer Moleküle zur Verfügung, insbesondere für die Anbindung von Verbindungen, die NH₂-, NH- oder OH-Gruppen enthalten. Überschüssige und ungebundene MSA-Copolymere werden vorzugsweise durch einen anschließenden Spülschritt in einem Lösungsmittel, in welchem die MSA-Copolymere gut löslich sind, entfernt. Besonders bevorzugt erfolgt die Spülung in einem organischen Lösungsmittel, insbesondere Aceton, für mindestens 15 min. Der Spülvorgang wird bevorzugt mindestens ein weiteres Mal wiederholt. Im Anschluss daran erfolgt vorzugsweise eine nochmalige Trocknung bei den Bedingungen, wie zuvor beschrieben.

Durch die nasschemische Behandlung wird die Oberfläche der dreidimensionalen Formkörper modifiziert, was in einer Gewichtszunahme des Formkörpers resultiert. Dreidimensionale Formkörper zeigen nach der nasschemischen Behandlung in einem erfindungsgemäßen Verfahren vorzugsweise eine Gewichtszunahme (Differenz des Gewichts nach nasschemischer Behandlung und des Gewichts vor der nasschemischen Behandlung) von 1 - 5 mg je cm³, insbesondere 1 - 2 mg je cm³.

Nasschemische Verfahren sind für alle porösen dreidimensionalen Formkörper geeignet, die zur erfindungsgemäßen Herstellung eines Zellkulturträgermaterials eingesetzt werden. Besonders bevorzugt wird eine Oberflächenmodifikation durch nasschemische Verfahren für dreidimensionale Formkörper eingesetzt, die eine Dicke von mehr als 1 cm aufweisen. Für den Fall, dass der Formkörper eine dreidimensionale Gitterstruktur ist, werden nasschemische Verfahren bevorzugt zur Oberflächenmodifikation für Gitterstrukturen mit vier und mehr Lagen länglicher Körper eingesetzt.

Im Anschluss an die Umwandlung der hydrophoben Oberfläche des elastischen, inerten Kunststoffs in eine hydrophile Oberfläche mit einem der Verfahrensschritte Plasmabehandlung, Beflammung oder Nasschemie wird darauf eine bioaktive Schicht aufgebracht, die mindestens ein bioaktives Protein enthält. Die hydrophile Oberfläche ist nicht dauerhaft stabil, so dass der sich der Behandlungsschritt zum Aufbringen der bioaktiven Schicht auf die hydrophile Oberfläche vorzugsweise unmittelbar an den vorherigen Behandlungsschritt zur Umwandlung der hydrophoben Oberfläche in eine hydrophile Oberfläche anschließt.

Zum Aufbringen der bioaktiven Schicht wird der poröse dreidimensionale Formkörper mit einer Flüssigkeit kontaktiert, die mindestens ein bioaktives Protein enthält. Die Flüssigkeit weist dabei vorzugsweise einen pH-Wert zwischen 2,5 und 4,5 auf, insbesondere zwischen 3 und 4, besonders bevorzugt pH 3,5. Die Flüssigkeit ist vorzugsweise eine wässrige Lösung. Vorzugsweise wird der Formkörper mit einer Suspension kontaktiert, in der das bioaktive Protein in einer wässrigen Flüssigkeit suspendiert vorliegt.

Bevorzugt wird der dreidimensionale Formkörper in einem Tauchverfahren mit der Flüssigkeit kontaktiert (Eintauchen in die Flüssigkeit). Der Tauchvorgang wird dabei vorzugsweise über mehrere Stunden, insbesondere mindestens 6 Stunden vollzogen.

Vorzugsweise wird das Zellkulturträgermaterial nach dem Aufbringen der bioaktiven Schicht, also nach dem Kontaktieren des dreidimensionalen Formkörpers mit der Flüssigkeit, die mindestens ein bioaktives Protein enthält, mindestens einmal mit einer wässrigen Lösung, bevorzugt Wasser, gespült. Vorzugsweise wird der Spülvorgang mehrmals durchgeführt. Besonders bevorzugt wird das Zellkulturträgermaterial nach dem Aufbringen der bioaktiven Schicht getrocknet. Dabei kann grundsätzlich jedes Verfahren zum Einsatz kommen, bei dem das bioaktive Protein nicht denaturiert. Geeignete Methoden sind das Trocknen an der Luft, vorzugsweise über längere Zeiträume, insbesondere mindestens 10 h, Trocknen unter erhöhten Temperaturen, im Exsikkator oder auch Gefriertrocknung.

Er verbleibt auf diese Weise eine bioaktive Schicht auf dem dreidimensionalen Formkörper, die im getrockneten Zustand eine maximale Dicke von 0,5 mm nicht überschreitet.

Das bioaktive Protein, das im erfindungsgemäßen Zellkulturträgermaterial enthalten ist, fördert das Anhaften der Zellen an der Oberfläche des Zellkulturträgermaterials. Dabei wird unter der "Bioaktivität" die Eigenschaft verstanden, dass Zellen durch deren Oberflächenproteine an die Proteine binden können. Bevorzugte bioaktive Proteine sind Proteine der extrazellulären Matrix oder Proteine, die dazu ähnliche Strukturen aufweisen. Besonders bevorzugt sind davon jedoch Proteine der extrazellulären Matrix.

Ist das bioaktive Protein welches in einem erfindungsgemäßen Verfahren auf der Oberfläche des dreidimensionalen Formkörpers aufgebracht wird ein Protein der extrazellulären Matrix, so ist dies vorzugsweise ein Protein aus der Familie der Kollagene. Besonders bevorzugt sind Kollagene vom Typ I, wobei auch Kollagene der übrigen Typen, insbesondere die fibrillären Kollagene der Typen II, III, V, und XI bevorzugt sind. Die Kollagene sind bevorzugt kurzfasrig und weisen eine Faserlänge zwischen 0,2 mm und 1 mm, insbesondere 0,3 mm und 0,5 mm auf. Besonders bevorzugt liegt das Kollagen als Suspension in einer wässrigen Lösung, insbesondere Wasser, mit einem Feststoffgehalt von maximal 5 Gewichts-%, vorzugsweise 1 bis 5 Gewichts-% vor. Ganz besonders bevorzugt davon sind Feststoffgehalte von maximal 2 Gewichts-%, insbesondere 1 bis 2 Gewichts-%.

Vorzugsweise wird mit einem erfindungsgemäßen Verfahren ein dreidimensionaler Formkörper, insbesondere ein dreidimensionales Gitter, welches Silikon enthält, durch Plasmabehandlung, Beflammung oder durch eine nasschemische Behandlung mit MSA-Copolymeren, behandelt. Im Anschluss daran wird der Formkörper, vorzugsweise nachdem dieser ggf. gespült und getrocknet wurde, durch Tauchen in eine wässrige Kollagen-haltige Lösung (vorzugsweise Kollagen l) oder eine Suspension aus einer wässrigen Flüssigkeit und Kollagen, insbesondere Kollagen I, behandelt. Dadurch bindet Kollagen an die Oberfläche des Silikons, so dass die Silikonoberfläche zur Besiedlung mit Zellen geeignet ist.

Eine bevorzugte Ausgestaltung des erfindungsgemäßen Verfahrens ist die Oberflächenmodifikation einer dreidimensionalen Gitterstruktur aus Silikon durch nasschemische Behandlung in einer vorzugsweise acetonhaltigen Lösung mit MSA-Copolymeren. Nach der nasschemischen Behandlung wird die Gitterstruktur getrocknet und mit einer Kollagen-I-Suspension in einer wässrigen Lösung mit einem Feststoffgehalt von maximal 2 Gewichts-% und einem pH-Wert zwischen 2,5 und 4,5 auf, insbesondere zwischen pH 3 und pH 4. Das Kollagen I ist dabei so ausgewählt, dass dessen Faserlänge in einem Bereich von 0,3 mm und 0,5 mm liegt. Zur Beschichtung mit Kollagen wird das nach der nasschemischen Behandlung getrocknete Gitter in die Kollagensuspension getaucht, so dass die Suspension die komplette Oberfläche der Gitterstruktur benetzt. Vorzugsweise wird die Kontaktierung durch Schütteln unterstützt, damit die Kollegensuspension auch in die inneren Strukturen des Gitters eindringen und diese vollständig benetzen kann. Der Schüttelvorgang wird nach einigen Minuten (vorzugsweise maximal 5 Minuten) gestoppt und die Gitterstruktur wird für mehrere Stunden, insbesondere mindestens 12 h, in der Kollagen-I-Suspension inkubiert. Nach Entnahme der Kollagen-I beschichteten Gitterstruktur wird eine Spülung und Desinfektion vorgenommen. Dies erfolgt vorzugsweise durch eine Spülung mit Wasser zu Beginn, wodurch die ungebundenen Kollagen-Fasern, die in den Poren des dreidimensionalen Formkörpers verbleiben, entfernt werden. Dadurch ist sichergestellt, dass die Poren des Formkörpers für die Besiedelung mit Zellen zur Verfügung stehen und nicht durch ungebundene Kollagenreste blockiert sind. Anschließend werden die Kollagen-I-beschichteten Gitterstrukturen für mehrere Stunden, insbesondere für mindestens 3 h, getrocknet. Im Anschluss daran erfolgt eine nochmalige Spülung mit Wasser und eine Trocknung für mindestens 10 h. Anschließend wird die Kollagen-I-beschichtete Gitterstruktur mit Ethanol desinfiziert und nach nochmaliger Trocknung für mindestens 10 h verpackt.

Eine weitere bevorzugte Ausgestaltung des erfindungsgemäßen Verfahrens ist die Oberflächenmodifikation einer maximal 1 cm dicken Gitterstruktur mit Plasmabehandlung oder Beflammung, besonders bevorzugt mittels Beflammung, zur Oberflächenmodifikation. Nach der Oberflächenmodifikation wird die Gitterstruktur getrocknet und mit einer Kollagen-I-Suspension in einer wässrigen Lösung mit einem Feststoffgehalt von maximal 2 Gewichts-% und einem pH-Wert zwischen 2,5 und 4,5 auf, insbesondere zwischen 3 und 4. Das Kollagen I ist dabei so ausgewählt, dass dessen Faserlänge in einem Bereich von 0,3 mm und 0,5 mm liegt. Zur Beschichtung mit Kollagen wird das Gitter in die Kollagensuspension getaucht, so dass die Suspension die komplette Oberfläche der Gitterstruktur benetzt. Vorzugsweise wird die Kontaktierung durch Schütteln unterstützt, damit die Kollegensuspension auch in die inneren Strukturen des Gitters eindringen und diese vollständig benetzen kann. Der Schüttelvorgang wird nach einigen Minuten (vorzugsweise maximal 5 Minuten) gestoppt und die Gitterstruktur wird für mehrere Stunden, insbesondere mindestens 12 h, in der Kollagen-I-Suspension inkubiert. Nach Entnahme der Kollagen-I beschichteten Gitterstruktur wird eine Spülung und Desinfektion vorgenommen. Dies erfolgt vorzugsweise durch eine Spülung mit Wasser zu Beginn, wodurch die ungebundenen Kollagen-Fasern, die in den Poren des dreidimensionalen Formkörpers verbleiben, entfernt werden. Anschließend werden die Kollagen-I-beschichteten Gitterstrukturen für mehrere Stunden, insbesondere für mindestens 3 h, getrocknet. Im Anschluss daran erfolgt eine nochmalige Spülung mit Wasser und eine Trocknung für mindestens 10 h. Anschließend wird die Kollagen-I-beschichtete Gitterstruktur mit Ethanol desinfiziert und nach nochmaliger Trocknung für mindestens 10 h verpackt.

Von der Erfindung umfasst ist auch ein elastisches Zellkulturträgermaterial mit einer porösen dreidimensionalen Struktur zur *in vitro* Kultivierung von adhärenten Zellen, dessen Oberfläche eine bioaktive Beschichtung mit einem bioaktiven Protein aufweist und welches durch ein erfindungsgemäßes Verfahren erhältlich ist.

Ferner umfasst die Erfindung auch ein elastisches Zellkulturträgermaterial mit einer porösen dreidimensionalen Struktur zur *in vitro* Kultivierung von adhärenten Zellen, dessen Oberfläche eine bioaktive Beschichtung mit einem bioaktiven Protein aufweist, das dadurch gekennzeichnet ist, dass das Zellkulturträgermaterial einen dreidimensionalen porösen Formkörper enthält, der mindestens einen elastischen, inerten Kunststoff enthält und der offenporig ist und ein interkonnektierendes Porensystem aufweist. Der dreidimensionale Formkörper ist ein dreidimensionales Gitter, das vorzugsweise aus mindestens zwei übereinanderliegenden parallelen Lagen aus jeweils mindestens zwei länglichen Körpern besteht, wobei die Lagen in einem Winkel von 1 bis 90° zueinander versetzt angeordnet sind, so dass sich die länglichen Körper der unterschiedlichen Lagen kreuzen. Auf der Oberfläche des elastischen, inerten Kunststoffs befindet sich eine bioaktive Schicht, die mindestens ein bioaktives Protein enthält. Die Zusammensetzung und Dimensionen des dreidimensionalen Formkörpers, der in einem erfindungsgemäßen Zellkulturträgermaterial enthalten ist, ist identisch zu den dreidimensionalen Formkörpern wie oben beschrieben, die in einem erfindungsgemäßen Verfahren zur Herstellung eines elastischen Zellkulturträgermaterials eingesetzt werden.

Das bioaktive Protein, das in der bioaktiven Schicht enthalten ist, ist vorzugsweise ein Protein der extrazellulären Matrix, insbesondere Kollagen, weiter bevorzugt ein fibrilläres Kollagen vom Typ I, II, III, V oder XI. Besonders bevorzugt ist das bioaktive Protein ein Kollagen vom Typ I. Besonders bevorzugt enthält die bioaktive Schicht ein kurzfasriges fibrilläres Kollagen mit einer Faserlänge zwischen 0,2 mm und 1 mm.

Die bioaktive Schicht, die sich auf dem dreidimensionalen Formkörper befindet, hat im getrockneten Zustand (also nach Trocknung des bioaktiv beschichteten erfindungsgemäßen Zellkulturträgermaterials) vorzugsweise eine Dicke, die maximal ein Viertel so groß ist, wie die Porengröße des dreidimensionalen Formkörpers. Bevorzugte Schichtdicken der bioaktiven Schicht sind maximal 0,5 mm, besonders bevorzugt maximal 0,3 mm. Besonders bevorzugt beträgt die Dicke der bioaktiven Schicht im getrockneten Zustand 0,1 mm bis 0,3 mm.

Die Erfindung umfasst auch ein Verfahren zur *in vitro* Kultivierung von Zellen, bei dem man
a) ein erfindungsgemäßes Zellkulturträgermaterial oder ein durch ein erfindungsgemäßes Verfahren erhältliches Zellkulturträgermaterial in einem Behältnis bereitgestellt wird,
b) die Zugabe von Zellen in einem Nährmedium erfolgt und
c) die Zellen unter für das Wachstum der Zellen zuträglichen Bedingungen kultiviert werden, so dass die Zellen auf der Oberfläche des Zellkulturträgermaterials adhärieren.

Die Porosität des Zellkulturträgermaterials ist so ausgewählt, dass optimale Wachstumsbedingungen für die zu kultivierenden Zellen herrschen. Bevorzugt werden primäre isolierte Zellen oder Zelllinien aus Gewebezellen, insbesondere aus Zellen der Leber, Niere, Muskeln, Nerven, Endothel oder Epithel oder Stammzellen kultiviert.

Das Nährmedium, das für die Kultivierung der Zellen ausgewählt wird und die Bedingungen der Kultivierung, insbesondere hinsichtlich Temperatur und Sauerstoffgehalt, entsprechen grundsätzlich den üblichen für die jeweiligen Zellen nach dem Stand der Technik eingesetzten Bedingungen. Es besteht jedoch aufgrund der im Vergleich zu zweidimensionalen Kulturen höheren Zelldichte in einem erfindungsgemäßen Kultivierungsverfahren ein erhöhter Nährstoffbedarf, so dass das Nährmedium ggf. öfters getauscht werden muss, als bei zweidimensionalen Kulturen. Durch den Einsatz des erfindungsgemäßen Zellkulturträgermaterials werden die Bedingungen für das dreidimensionale Wachstum der Zellen verbessert, so dass eine höhere volumenbezogene Zellausbeute erzielt wird.

Die Erfindung umfasst weiterhin die Verwendung eines erfindungsgemäßen Zellkulturträgermaterials oder eines Zellkulturträgermaterials, welches durch ein erfindungsgemäßes Verfahren erhältlich ist, zur *in vitro* Kultivierung von Zellen.

Durch die Verwendung eines elastischen, inerten Kunststoffs als Hauptbestandteil des dreidimensionalen Formkörpers, der die Grundstruktur des Zellkulturträgermaterials vorgibt, ist das Zellkulturträgermaterial besonders robust. Durch die Elastizität ist es äußerst vielseitig und flexibel einsetzbar. So kann ein erfindungsgemäßes komplexes dreidimensionales Zellkulturträgermaterial auch durch enge Öffnungen von Zellkulturgefäßen eingebracht werden und kann im Inneren des Gefäßes seine ursprüngliche Form wieder entfalten.

Der eingesetzte dreidimensionale Formkörper ist wiederverwendbar und kann nach einer Anwendung zur *in vitro* Kultivierung von Zellen nach Reinigung und Sterilisation erneut mit einer bioaktiven Schicht überzogen werden. Die in dem dreidimensionalen Formkörper eingesetzten Materialien sind allesamt biokompatibel, so dass das Zellkulturträgermaterial selbst keinen negativen oder toxischen Einfluss auf die zu kultivierenden Zellen ausübt. Die Materialien, die in dem dreidimensionalen Formkörper enthalten sind, sind vorzugsweise alle sterilisierbar. In diesem Fall kann der Formkörper sterilisiert werden und verbleibt auch in den anschließenden Behandlungsschritten (Umwandlung der hydrophoben in eine hydrophile Oberfläche und Aufbringen der bioaktiven Schicht), die dann in steriler Umgebung durchgeführt werden, steril. So können Zellkulturträgermaterialien hergestellt werden, die problemlos in einer Langzeit- oder Dauerkultur eingesetzt werden können. Dadurch, dass die erfindungsgemäßen Zellkulturträgermaterialien eine definierte Porosität aufweisen, die je nach dem zu kultivierenden Zelltyp ausgewählt werden kann, können optimale Wachstumsbedingungen geschaffen werden. Die Wachstumsoberfläche bei erfindungsgemäßen dreidimensionalen Zellkulturträgermaterialien ist bezogen auf das Volumen, in dem die Zellkultur durchgeführt wird signifikant höher, als die von zweidimensionalen Zellkulturen.

Anhand folgender Figuren und Ausführungsbeispiele soll die Erfindung näher erläutert werden, ohne die Erfindung auf diese zu beschränken.
Fig. 1 bis Fig. 6 zeigen dreidimensionale Gitterstrukturen erfindungsgemäßer Zellkulturträgermaterialien. Die länglichen Körper weisen eine runde Querschnittsfläche auf. Die Lagen der länglichen Körper sind dabei so angeordnet, dass sich die länglichen Körper einer Lage mit mindestens einer der anliegenden Lagen überkreuzen.
Fig. 1 und Fig. 2 zeigen dreidimensionale Gitterstrukturen mit zwei Lagen länglicher Körper. Fig. 3, Fig. 4 und Fig. 6 zeigen schematische Darstellungen dreidimensionaler Gitterstrukturen mit vier Lagen länglicher Körper. Es sind jeweils zwei gleichartige Lagen vorhanden, die schematisch getrennt dargestellt sind. In der dreidimensionalen Gitterstruktur liegen die Lagen aufeinander auf.

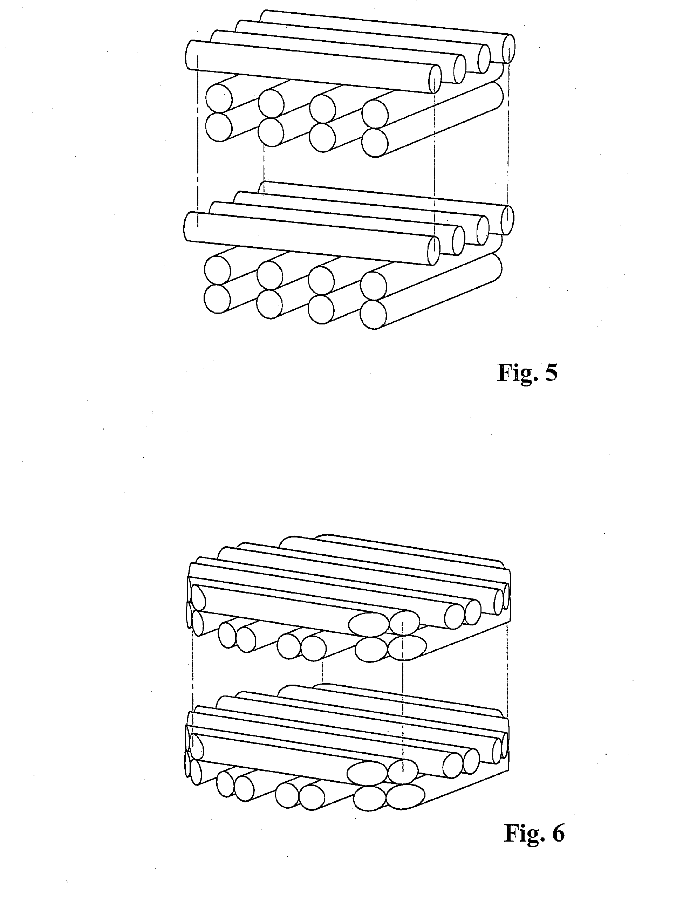

Fig. 5 zeigt eine exemplarische dreidimensionale Gitterstruktur mit sechs Lagen länglicher Körper. Es sind jeweils zwei gleichartige Lagen vorhanden, die schematisch getrennt dargestellt sind. In der dreidimensionalen Gitterstruktur liegen die Lagen aufeinander auf.

### Ausführungsbeispiel 1: Behandlung der Oberfläche einer dreidimensionalen Gitterstruktur aus Silikon mit Plasma

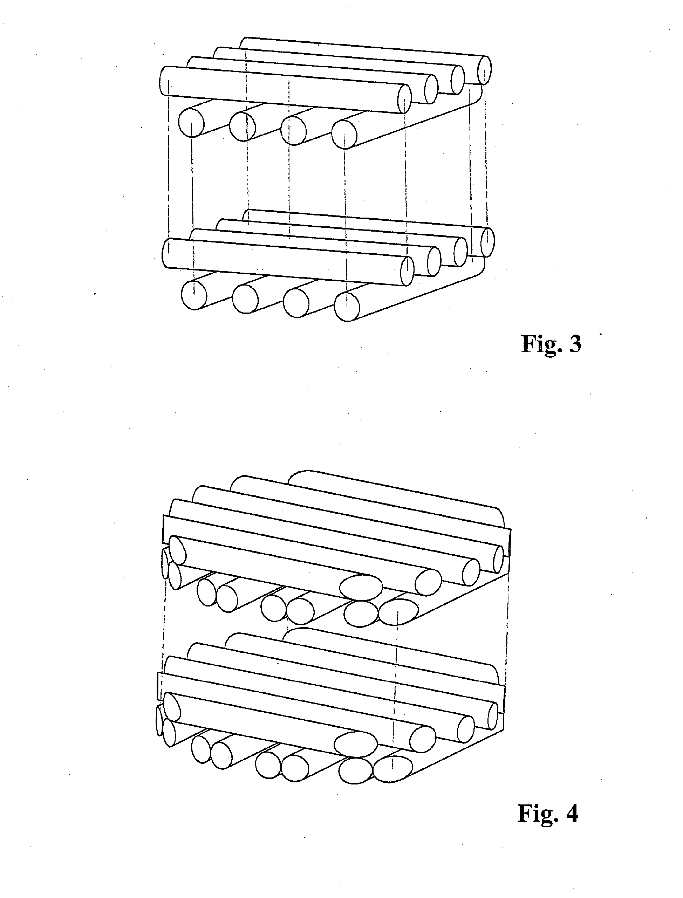

Es wurde eine dreidimensionale Gitterstruktur, wie sie in Fig. 3 abgebildet ist, mittels Rapid Prototyping hergestellt. Die Struktur enthält vier Lagen mit parallelen länglichen Stäben. Zur Oberflächenmodifikation, durch die die hydrophobe Oberfläche des Silikons in eine hydrophile Oberfläche umgewandelt wird, so dass Kollagen angebunden werden kann, wurde eine Plasmabehandlung durchgeführt. Dafür wurde die Gitterstruktur zunächst in Ethanol gespült, getrocknet und in die Plasmakammer eingebracht. Es wurde für 5 min eine Plasmabehandlung unter Argon durchgeführt. Nach Beendigung der Plasmabehandlung wurde die Gitterstruktur aus der Plasmakammer entnommen und unmittelbar danach in eine Kollagen-I-Suspension getaucht.

### Ausführungsbeispiel 2: Behandlung der Oberfläche einer dreidimensionalen Gitterstruktur aus Silikon mit Beflammung

Es wurde eine dreidimensionale Gitterstruktur, wie sie in Fig. 3 abgebildet ist, mittels Rapid Prototyping hergestellt. Die Struktur enthielt vier Lagen mit parallelen länglichen Stäben. Zur Oberflächenmodifikation, durch die die hydrophobe Oberfläche des Silikons in eine hydrophile Oberfläche umgewandelt wird, so dass Kollagen angebunden werden kann, wurde eine Beflammung durchgeführt. Dafür wurde die Gitterstruktur zunächst in Ethanol gespült, getrocknet und anschließend für maximal 1 Sekunde mit einem entzündeten Propan-Butan-Gemisch beflammt. Dies wurde zunächst von einer Seite durchgeführt (Fig. 3 von oben), anschließend wurde die Gitterstruktur gedreht und es erfolgte eine nochmalige Beflammung der anderen Seite der Gitterstruktur. Auf diese Weise wurde sichergestellt, dass die komplette Oberfläche der Gitterstruktur hydrophiliert ist. Im direkten Anschluss daran wurde die Gitterstruktur in eine Kollagen-I-Suspension getaucht.

### Ausführungsbeispiel 3: Behandlung der Oberfläche einer dreidimensionalen Gitterstruktur aus Silikon mit Nasschemie

Es wurde eine dreidimensionale Gitterstruktur, wie sie in Fig. 5 abgebildet ist, mittels Rapid Prototyping hergestellt. Die Struktur enthält sechs Lagen mit parallelen länglichen Stäben. Zur Oberflächenmodifikation, durch die die hydrophobe Oberfläche des Silikons in eine hydrophile Oberfläche umgewandelt wird, so dass Kollagen angebunden werden kann, wurde eine nasschemische Behandlung durchgeführt. Dafür wurde die Gitterstruktur zunächst in Aceton gespült und getrocknet.

Alternierende Copolymere aus Maleinsäureanhydrid und Allyltrimethylsilan (AMTS/MSA-Copolymere) mit einem Molekulargewicht von 4000 - 10000 g/mol wurden in Aceton gelöst. Niedermolekulare Polysiloxane mit einem Molekulargewicht von 3000 - 10000 g/mol (Silikonöle) wurden in Aceton aufgenommen, so dass die Lösung eine Viskosität weniger als 100 centistoke aufweist. Zur Anbindung der AMTS/MSA-Copolymere an die Silikonoberfläche wurde die Gitterstruktur zunächst für 30 Minuten in der Lösung mit den Polysiloxanen inkubiert. Nach der Quellzeit wurden sie aus dem Bad mit den Polysiloxanen entnommen, mit Zellstoff abgetupft und gewogen. Danach wurden die Gitterstrukturen für 24 h bei Raumtemperatur gelagert, nochmals abgetupft und gewogen.

Zum Anbinden der ATMS/MSA-Copolymere auf der Silikonoberfläche wurden die Gitterstrukturen für 30 min in eine 10 %-ige (v/v) Lösung der ATMS/MSA-Copolymere in Aceton getaucht. Anschließend erfolgt eine Trocknung bei 50 °C im Trockenschrank über 3 Stunden. Danach erfolgte die Lagerung in Aceton (2 x 30 min), um ungebundene Anteile der ATMS/MSA-Copolymere zu entfernen. Nach 30 min wurde das Aceton erneuert. Nach der anschließenden Trocknung bei 50 °C im Trockenschrank über 8 h wurden die Probekörper gewogen. Es wurde eine Gewichtszunahme von 1 bis 2 mg je cm² festgestellt.

Anschließend kann die Gitterstruktur in eine Kollagen-I-Suspension getaucht werden.

### Ausführungsbeispiel 4: Kollagenbeschichtung

Oberflächenmodifizierte Gitterstrukturen aus Silikon aus Ausführungsbeispiel 1, 2 und 3 wurden mit Kollagen-I beschichtet. Dafür wurden die Gitterstrukturen in eine 1-%ige (Gewichts-%) Kollagen-I-Suspension getaucht. Die Suspension enthielt Kollagen-I mit einer Faserlänge von 0,4 mm, die in einer wässrigen Lösung bei einem pH-Wert von 3,5 suspendiert war. Zur Beschichtung mit Kollagen wurde das Gitter in die Kollagensuspension getaucht, so dass die Suspension die komplette Oberfläche der Gitterstruktur benetzte. Zunächst wurde die Kontaktierung durch Schütteln unterstützt, damit die Kollegensuspension auch in die inneren Strukturen des Gitters eindringen und diese vollständig benetzen konnte. Der Schüttelvorgang wurde nach 5 Minuten gestoppt und die Gitterstruktur wurde für 24 Stunden in der Kollagen-I-Suspension inkubiert. Nach Entnahme der Kollagen-I beschichteten Gitterstruktur wurde eine Spülung und Desinfektion vorgenommen. Es erfolgte zunächst eine Spülung mit Reinstwasser, wodurch die überschüssigen Kollagen-Fasern entfernt wurden. Anschließend wurden die Kollagen-I-beschichteten Gitterstrukturen für vier Stunden getrocknet. Im Anschluss wurde nochmals mit Reinstwasser gespült und das Gitter für 12 Stunden getrocknet. Abschließend wurde die Kollagen-I-beschichtete Gitterstruktur mit Ethanol desinfiziert und nach nochmaliger Trocknung für 12 h schließlich verpackt und gelagert.

Die bioaktive Beschichtung des dreidimensionalen Formkörpers weist eine Dicke von 0,2 - 0,3 mm auf.

## Patentansprüche

1. Verfahren zur Herstellung eines elastischen Zellkulturträgermaterials mit einer porösen dreidimensionalen Struktur, welches zur *in vitro* Kultivierung von Zellen geeignet ist und ein dreidimensionales Wachstum von Zellen ermöglicht, **gekennzeichnet durch** die folgenden Schritte:
a) Bereitstellung eines porösen dreidimensionalen Formkörpers mittels Rapid Prototyping, wobei der Formkörper ein dreidimensionales Gitter ist, das aus mindestens zwei übereinanderliegenden parallelen Lagen aus jeweils mindestens zwei länglichen Körpern besteht, wobei die Lagen in einem Winkel von 1 bis 90° zueinander versetzt angeordnet sind, so dass sich die länglichen Körper der unterschiedlichen Lagen kreuzen, wobei der Formkörper offenporig ist und ein interkonnektierendes Porensystem aufweist, und wobei der Formkörper mindestens einen elastischen, inerten Kunststoff mit einer hydrophoben Oberfläche enthält,
b) Behandlung der Oberfläche des porösen dreidimensionalen Formkörpers, so dass die hydrophobe Oberfläche des in dem Formkörper enthaltenen elastischen, inerten Kunststoffs in eine hydrophile Oberfläche umgewandelt wird,
c) Aufbringen einer bioaktiven Schicht auf die hydrophile Oberfläche des elastischen, inerten Kunststoffs des Formkörpers **durch** Kontaktieren des Formkörpers mit einer Flüssigkeit, welche mindestens ein bioaktives Protein enthält.

2. Verfahren nach Anspruch 1, **dadurch gekennzeichnet, dass** die Porengröße des dreidimensionalen Formkörpers zwischen 0,4 mm und 1 mm beträgt.

3. Verfahren nach einem der Ansprüche 1 bis 2, **dadurch gekennzeichnet, dass** die Behandlung der Oberfläche durch Plasmabehandlung, Beflammung oder durch nasschemische Behandlung in einer Lösung mit Copolymeren und/oder Terpolymeren aus Maleinsäureanhydrid und mindestens einem Vinylmonomer durchgeführt wird.

4. Verfahren nach einem der Ansprüche 1 bis 3, **dadurch gekennzeichnet, dass** der elastische, inerte Kunststoff Silikon ist.

5. Verfahren nach einem der Ansprüche 1 bis 4, **dadurch gekennzeichnet, dass** die Flüssigkeit, welche mindestens ein bioaktives Protein enthält, einen pH-Wert von 2,5 bis 4,5 aufweist.

6. Verfahren nach einem der Ansprüche 1 bis 5, **dadurch gekennzeichnet, dass** das bioaktive Protein Kollagen ist.

7. Verfahren nach Anspruch 6, **dadurch gekennzeichnet, dass** das Kollagen eine Faserlänge zwischen 0,2 mm und 1 mm aufweist.

8. Verfahren nach Anspruch 6 oder 7, **dadurch gekennzeichnet, dass** das Kollagen als Suspension in Wasser mit einem Feststoffgehalt von maximal 2 Gewichts-% vorliegt.

9. Verfahren nach Anspruch 8, **dadurch gekennzeichnet, dass** der dreidimensionale Formkörper ein dreidimensionales Gitter aus Silikon ist, das aus mindestens zwei übereinanderliegenden parallelen Lagen aus jeweils mindestens zwei länglichen Körpern besteht, wobei die Lagen in einem Winkel von 1 bis 90° zueinander versetzt angeordnet sind, so dass sich die länglichen Körper der unterschiedlichen Lagen kreuzen.

10. Elastisches Zellkulturträgermaterial mit einer porösen dreidimensionalen Struktur zur *in vitro* Kultivierung von adhärenten Zellen, dessen Oberfläche eine bioaktive Beschichtung mit einem bioaktiven Protein aufweist, **dadurch gekennzeichnet, dass** das Zellkulturträgermaterial einen dreidimensionalen porösen Formkörper enthält, wobei der Formkörper ein dreidimensionales Gitter ist, das aus mindestens zwei übereinanderliegenden parallelen Lagen aus jeweils mindestens zwei länglichen Körpern besteht, wobei die Lagen in einem Winkel von 1 bis 90° zueinander versetzt angeordnet sind, so dass sich die länglichen Körper der unterschiedlichen Lagen kreuzen, wobei der Formkörper mindestens einen elastischen, inerten Kunststoff enthält, wobei der Formkörper offenporig ist und ein interkonnektierendes Porensystem aufweist, wobei sich auf der Oberfläche des elastischen, inerten Kunststoffs eine bioaktive Schicht befindet, die mindestens ein bioaktives Protein enthält.

11. Zellkulturträgermaterial nach Anspruch 10, **dadurch gekennzeichnet, dass** das bioaktive Protein ein Protein der extrazellulären Matrix, insbesondere Kollagen, ist.

12. Zellkulturträgermaterial nach einem der Ansprüche 10 oder 11, **dadurch gekennzeichnet, dass** der elastische, inerte Kunststoff Silikon ist.

13. Zellkulturträgermaterial nach einem der Ansprüche 10 bis 12, **dadurch gekennzeichnet, dass** der dreidimensionale Formkörper ein dreidimensionales Gitter aus Silikon ist, dessen Oberfläche eine bioaktive Beschichtung mit Kollagen aufweist, wobei die Faserlänge des Kollagens zwischen 0,2 mm und 1 mm beträgt.

14. Verfahren zur *in vitro* Kultivierung von Zellen, bei dem:
a) ein Zellkulturträgermaterial nach einem der Ansprüche 10 bis 13 oder ein durch ein Verfahren nach einem der Ansprüche 1 bis 9 erhältliches Zellkulturträgermaterial in einem Behältnis bereitgestellt wird,
b) die Zugabe von Zellen in einem Nährmedium erfolgt und
c) die Zellen unter für das Wachstum der Zellen zuträglichen Bedingungen kultiviert werden, so dass die Zellen auf der Oberfläche des Zellkulturträgermaterials adhärieren.

15. Verwendung eines Zellkulturträgermaterials nach einem der Ansprüche 10 bis 13 oder eines durch ein Verfahren nach einem der Ansprüche 1 bis 9 erhältlichen Zellkulturträgermaterials zur *in vitro* Kultivierung von Zellen.
